# EUROPEAN PATENT SPECIFICATION

(11) **EP 3 817 574 B1**
(45) Date of publication and mention of the grant of the patent: **03.09.2025**
(21) Application number: 19734418.7
(22) Date of filing: 03.07.2019
(51) Int. Cl.: A23L 11/00, A23L 2/38, A23L 2/66, A23L 29/00, A23L 33/185, A23J 1/00, A23J 1/14, A23L 2/39, A23L 11/60, A23L 11/65

(54) **FOOD COMPOSITION COMPRISING PEA PROTEINS AND A POTASSIUM METAPHOSPHATE**
NAHRUNGSMITTELZUSAMMENSETZUNG MIT ERBSENPROTEINEN UND KALIUMMETAPHOSPHAT
COMPOSITION ALIMENTAIRE COMPRENANT DES PROTÉINES DE POIS ET UN MÉTAPHOSPHATE DE POTASSIUM

(30) Priority: 03.07.2018 EP 18305869
(43) Date of publication of application: 12.05.2021
(73) Proprietor: ROQUETTE FRERES, 62136 Lestrem (FR)
(72) Inventor: ITO, Goichi, Koganei-city Tokyo Tokyo 184-0014 (JP)
(74) Representative: Plasseraud IP
(86) International application number: PCT/EP2019/067901
(87) International publication number: WO 2020/007940

(56) References cited:
- CN-A- 1 813 576
- JP-A- 2001 204 443
- JP-A- 2017 171 593
- US-A- 3 637 643
- US-A1- 2009 117 231

## Description

### FIELD OF THE INVENTION

The present invention relates to a food composition, in particular a powder mix for beverages, based on pea proteins, having improved organoleptic properties, in particular a reduced sandy mouthfeel, to a process in order to obtain said composition, and also to the uses thereof, in particular in the food-processing field and most particularly the preparation of food formulations.

### PRIOR ART

Along with carbohydrates and lipids, proteins constitute a significant part of our diet. Daily protein requirements are generally between 12% and 20% of food intake.

Generally, consumed proteins are either of animal origin (referred to as animal proteins), for instance meat, fish, eggs and milk products, or of plant origin (referred to as plant proteins), for instance cereals, oleaginous plants and leguminous plants.

In industrialized countries, protein intakes are mainly in the form of animal proteins. However, many studies demonstrate that excessive consumption of animal proteins to the detriment of plant proteins is one of the causes of increase of cancer and cardiovascular diseases.

Moreover, animal proteins have many disadvantages, both in terms of their allergenicity, in particular proteins from milk or eggs, and environmental impact, in particular the damaging effects of intensive farming.

Thus, as an alternative, manufacturers have gradually turned to plant proteins. Indeed, it is known practice to use plant proteins in order to replace all or some of the animal proteins in foods.

Such a replacement is not always easy because plant proteins have functional properties that are different from those of animal proteins. These functional properties can be physical or physicochemical properties which have an effect on the sensory qualities of food compositions generated during technological transformations, storage or domestic culinary preparations.

Among plant proteins, it is for example known practice to use leguminous-plant proteins. Compared to milk proteins, they exhibit a strong nutritional advantage but their high cost constitutes an impediment to their large-scale use in the food-processing field. Thus, milk proteins cannot be easily replaced with leguminous-plant proteins.

One drawback of certain plant proteins, in particular leguminous-plant proteins, is that they don't behave like milk proteins, particularly in terms of texture, especially as they give a sandy feeling. This drawback is especially challenging in the formulation of powder-mix beverages.

Powder-mix protein based compositions are often difficult to dissolve in cold water, and can further exhibit a sandy mouthfeel. The sandy mouthfeel feels artificial and is a strong brake for consumers to accept premium vegetal-based powder beverages.

One solution to this problem is to grind the protein powder in order to lower its granulometry. The obtained protein powder will dissolve better and no sandy mouthfeel will be detected by consumer. However, the supplemental grinding operation unit is expensive and complex to manage.

A second solution is to dissolve the powder in hot water. However, this increases the length of the rehydration process. As powder based beverages are often reconstituted by consumers, this drawback is unacceptable, as consumers want a quick and simple way to prepare their beverages. JP2017171593 discloses a method for preparing a nutritional composition comprising mixing soybean protein with sodium metaphosphate and potassium metaphosphate. US2009/117231 and US3637643 disclose rehydratable compositions comprising soybean protein and potassium hexametaphosphate or sodium hexametaphosphate and potassium polymetaphosphate. CN1813576 discloses a mixture of hydrolysed plant protein and potassium metaphosphate.

Thus, in order to promote the replacement of animal proteins with plant proteins in the food processing industry, there is still a need for a solution which makes it possible to improve, in a simple way, the texture, in particular to decrease the sandy mouthfeel, of plant protein-based food compositions, in particular leguminous-plant protein-based food compositions, more particularly pea protein-based food compositions.

The Applicant has thus, to its credit, developed a process which makes it possible to obtain a composition comprising pea proteins, said composition having an improved texture, in particular a reduced sandy mouthfeel, compared to the compositions of the prior art.

### SUMMARY OF THE INVENTION

A first object of the present invention is a rehydratable food composition comprising pea proteins, and a potassium metaphosphate.

In one embodiment, said rehydratable food composition further comprises a sodium metaphosphate.

The rehydratable food composition of the invention exhibits improved organoleptic properties, in particular a reduced sandy mouthfeel, compared to a composition comprising plant proteins only.

A second object of the present invention is a process for preparing the rehydratable food composition of the invention, said process comprising mixing pea proteins, and a potassium metaphosphate.

In one embodiment, the first step of the process is preparing an aqueous or solid composition comprising pea proteins, and a combination of a sodium metaphosphate and a potassium metaphosphate.

The present invention also relates to a rehydratable food composition based on pea proteins, which can be obtained by the process according to the invention.

Finally, another object of the present invention is the use of said rehydratable food composition in the food-processing industry, and more particularly for preparing food formulations.

### DETAILED DESCRIPTION

The rehydratable food composition of the present invention comprises pea proteins and a potassium metaphosphate.

The present disclosure not encompassed by the claims relates to a rehydratable food composition comprising plant proteins, preferably leguminous plant proteins, and a potassium metaphosphate.

For the purposes of the present invention, "food composition" is intended to mean a composition that can be ingested by an animal or a human being. Examples of food compositions include foodstuffs for human consumption, animal feed and beverages. The term "rehydratable food composition" intends to mean a solid food composition that may be rehydrated, i.e. by addition of water, before being consumed.

For the purposes of the present invention, "organoleptic properties" is intended to mean aspects of a composition that a person experiences via the senses - including taste, sight, smell, and touch.

The plant proteins introduced in the composition of the invention are pea proteins.

In a preferred embodiment, the term "plant protein(s)" is to be understood as a native plant protein(s), which has not been subjected to a modification treatment by chemical and/or enzymatic hydrolysis. Such chemical and/or enzymatic hydrolysis is carried out with the aim of reducing the molecular weight of the plant protein(s). It has to distinguished from minor hydrolysis that may occur in conventional protein extraction processes.

For the purposes of the present invention, the term "leguminous plant" is intended to mean any plant belonging to the families Caesalpiniaceae, Mimosaceae or Papilionaceae, such as alfalfa, clover, lupin, pea, bean, broad bean, horse bean or lentil, and more particularly pea. The term "leguminous plant proteins" is intended to mean proteins that are derived from a leguminous plant, for example by extraction and optionally further modification.

The term "pea" is herein considered in its broadest accepted sense and includes in particular:
- all varieties of "smooth pea" and of "wrinkled pea", and
- all mutant varieties of "smooth pea" and of "wrinkled pea", this being whatever the uses for which said varieties are generally intended (food for human consumption, animal feed and/or other uses).

In the present application, the term "pea" includes the varieties of pea belonging to the *Pisum* genus and more particularly *Pisum sativum.*

Said mutant varieties are in particular those known as "r mutants", "rb mutants", "rug 3 mutants", "rug 4 mutants", "rug 5 mutants" and "lam mutants" as described in the article by C-L HEYDLEY et al. entitled "Developing novel pea starches", Proceedings of the Symposium of the Industrial Biochemistry and Biotechnology Group of the Biochemical Society, 1996, pp.77-87.

In a preferred embodiment, said leguminous-plant protein is derived from smooth pea.

Peas are leguminous plants with protein-rich seeds which have been the widely developed in Europe and in France since the 1970s, not only as a protein source for animal feed, but also as food for human consumption.

Like all leguminous-plant proteins, pea proteins consist of three main classes of proteins: globulins, albumins and "insoluble" proteins.

The value of pea proteins lies in their good emulsifying capacities, their lack of allergenicity and their low cost, which makes them an economical functional ingredient.

Furthermore, pea proteins favourably contribute to sustainable development and their carbon impact is very positive. This is because pea cultivation is environmentally friendly and does not require nitrogenous fertilizers, since the pea fixes atmospheric nitrogen.

For the purposes of the present invention, the term "composition with reduced sandy mouthfeel" is intended to mean a composition for which the perception of a sandy mouthfeel is decreased, or even completely masked compared to a composition comprising pea proteins only.

The rehydratable food composition of the invention comprises pea proteins, and a potassium metaphosphate.

In particular, the rehydratable food composition may be a dry powder. Said dry powder may be used as a base for reconstitution of a beverage by addition of a liquid or a solution, e.g. water. Such beverages may be chosen from the group consisting of:
- beverages intended for dietetic nutrition,
- beverages intended for the nutrition of sportsmen and sportswomen,
- beverages intended for infant nutrition,
- beverages intended for clinical nutrition and/or for individuals suffering from undernourishment,
- beverages intended for the nutrition of the elderly.

As used herein, the term "potassium metaphosphate" refers to a potassium salt of metaphosphoric acid (HPO₃), a dimer, trimer, tetramer, hexamer or polymer thereof, and mixtures thereof. Also encompassed by said term are the hydrated and partially hydrated forms thereof. Potassium metaphosphate can also be referred to as potassium polymetaphosphate, potassium polyphosphate and Korrol salt. Potassium metaphosphate may be referenced under CAS number 7790-53-6 or 12764-57-7. In one embodiment, potassium metaphosphate refers to a compound of formula (KPO₃), wherein n is from 2 to 20,000, preferably n is from 5,000 to 15,000, more preferably n is from 8,000 to 12,000. In another embodiment, potassium metaphosphate refers to a heterogeneous mixture of potassium salts of linear condensed polyphosphoric acids of general formula Hₙ₊₂PₙO₃ₙ₊₁ where n is from 2 to 20,000, preferably n is from 5,000 to 15,000, more preferably n is from 8,000 to 12,000.

In a preferred embodiment, the amount of potassium metaphosphate in the composition may be from 0.008% to 33%, preferably from 0.01% to 20%, more preferably from 0.025% to 8%, by weight based on the dry weight of the composition.

The rehydratable food composition of the invention may further comprise a sodium metaphosphate.

As used herein, the term "sodium metaphosphate" refers to a sodium salt of metaphosphoric acid (HPO₃), a dimer, trimer, tetramer, hexamer or polymer thereof, and mixtures thereof. Also encompassed by said term are the hydrated and partially hydrated forms thereof. Sodium metaphosphate can also be referred to as sodium trimetaphosphate, sodium tetrametaphosphate, sodium hexametaphosphate, sodium polyphosphate and Graham's salt. Sodium metaphosphate may be referenced under CAS number 68915-31-1, 7785-84-4, 10124-56-8, 10361-03-2 or 50813-16-6. In one embodiment, sodium metaphosphate refers to a compound of formula (NaₙH₂PₙO₃ₙ₊₁) wherein n is from 1 to 1,000, preferably n is from 5 to 100, more preferably n is 10 to 50. In another embodiment, sodium metaphosphate refers to a mixture of several amorphous, water-soluble polyphosphates composed of linear chains of metaphosphate units (NaPO₃)ₓ terminated by Na₂PO₄ groups wherein x is from 1 to 1,000, preferably x is from 5 to 100, more preferably x is 10 to 50. In particular, the Na₂O/P₂O₅ ratio may vary from about 1.3 for sodium tetrapolyphosphate (x = 4); to about 1.1 for Graham's salt, commonly called sodium hexametaphosphate (x = 13 to 18); and to about 1.0 for the higher molecular weight sodium polyphosphates (x = 20 to 100 or more). In another embodiment, sodium metaphosphate refers to a heterogeneous mixture of sodium salts of linear condensed polyphosphoric acids of general formula Hₙ₊₂PₙO₃ₙ₊₁ where n is from 2 to 1,000, preferably n is from 5 to 100, more preferably n is from 10 to 50.

The total amount of sodium metaphosphate and potassium metaphosphate in the composition may be from 0.01% to 8%, preferably from 0.02% to 6%, more preferably from 0.025% to 5%, by weight based on the dry weight of the composition.

Further, the weight percentage of potassium metaphosphate based on the total amount of potassium metaphosphate and sodium metaphosphate in the composition may be from 2% to 80%, preferably from 5% to 60%, even more preferably from 10% to 50%.

The rehydratable food composition of the invention may exhibit a rapid dissolution time, in particular less than 1 minute. The dissolution time may be, for example, visually assessed by shaking the composition in water at ambient temperature, i.e. at 20-25°C until complete dissolution.

The rehydratable food composition of the invention may be obtained with a process comprising mixing pea proteins, and a potassium metaphosphate.

The composition obtained by mixing the pea proteins and the potassium metaphosphate according to the process of the invention may be in the form of a solid composition, for example a solid powder mix, or an aqueous composition, for example an aqueous solution or suspension.

In a preferred embodiment, the process comprises preparing a composition comprising pea proteins, and a combination of a sodium metaphosphate and a potassium metaphosphate.

The present disclosure not encompassed by the claims relates to a process comprising preparing a composition comprising plant proteins, preferably leguminous plant proteins, more preferably pea proteins, and a combination of a sodium metaphosphate and a potassium metaphosphate.

Said process can be carried out according to the common practices of those skilled in the art. For example, the process can comprise mixing, on the one hand, an aqueous solution or suspension comprising leguminous-plant proteins with, on the other hand, an aqueous solution comprising a potassium metaphosphate or a combination of a sodium metaphosphate and a potassium metaphosphate.

In this case, the solution may further be homogenized, heat sterilized and dried. In particular, the homogenization may be carried at high pressure, for example at a pressure of between 3 MPa and 100 MPa, in particular between 15 MPa and 50 MPa, and most particularly at approximately 20 MPa.

The homogenized or non-homogenized aqueous solution or suspension may be subjected to a heat-sterilization step

In general, heat sterilization can be carried out by heating the composition, for example at a temperature greater than 100°C, for a period of time sufficient to inhibit the enzymes and any form of microorganisms, in particular sporulating bacteria. The sterilization may be carried out at high temperature, that is to say a temperature of 135°C to 150°C, for a period usually not exceeding 15 seconds, which corresponds to UHT (Ultra-High Temperature) sterilization. This technique has the advantage of preserving the nutritional and organoleptic properties of the sterilized product.

The heat-sterilization step can be carried out by means of the devices and techniques known to those skilled in the art. The heat-sterilized aqueous solution or suspension comprising leguminous-plant proteins and a potassium metaphosphate or a combination of a sodium metaphosphate and a potassium metaphosphate may be further subjected to drying which can be done by well-known technology like spray-drying.

In another embodiment, the process can comprise mixing directly dry pea proteins and a dry potassium metaphosphate or a combination of a dry sodium metaphosphate and a dry potassium metaphosphate. The dry mixing can be done using well-known apparatus from the state of the art. In this case, the dry powder composition of the invention can be directly packed and sold.

The mixing step may be advantageously carried out in such a way as to obtain a composition with the previously defined weight percentages of potassium metaphosphate or combination of sodium metaphosphate and potassium metaphosphate based on the dry weight of the composition.

The leguminous-plant protein introduced in the process of the invention is as defined above. In the present disclosure not encompassed by the claims, said leguminous-plant protein is chosen from the group consisting of bean, broad bean and horse bean, and mixtures thereof. In the present invention, said leguminous-plant protein is derived from pea.

In a preferred embodiment, the process of the invention may further comprise the addition of one or more additives to said composition.

The additive(s) can in particular be chosen from gelling agents, soluble plant fibres, sugar, vegetable oils, polysaccharides, sodium chloride, emulsifying agents, food dyes, preservatives, sweeteners and thickeners. Preferably, the additives are chosen from gelling agents, soluble plant fibres, sugar, vegetable oils, polysaccharides, sodium chloride and emulsifying agents.

Preferably, the gelling agent is chosen from alginates, agar agar, carrageenans, arabinogalactan, gellan gum, gelatin and pectins. Preferably, the gelling agent is gellan gum.

Preferably, the soluble plant fibre is chosen from the group consisting of fructans including fructooligosaccharides (FOSs) and inulin, glucooligosaccharides (GOSs), isomaltooligosaccharides (IMOs), trans-galactooligosaccharides (TOSs), pyrodextrins, polydextrose, branched maltodextrins, indigestible dextrins and soluble oligosaccharides derived from oleaginous or protein-producing plants.

The term "soluble fibre" is intended to mean water-soluble fibres. The fibres can be quantitatively determined according to various AOAC methods. Mention may be made, by way of example, of AOAC methods 997.08 and 999.03 for fructans, FOSs and inulin, AOAC method 2000.11 for polydextrose, AOAC method 2001.03 for quantitatively determining the fibres contained in branched maltodextrins and indigestible dextrins, or AOAC method 2001.02 for GOSs and also soluble oligosaccharides derived from oleaginous or protein-producing plants.

Advantageously, the soluble plant fibre is obtained from partially hydrolysed wheat or corn starch, and contains up to 85% of total fibre.

Preferably, the vegetable oil is chosen from groundnut, avocado, borage, camelina, safflower, hemp, rapeseed, wheat germ, linseed, nigella, hazelnut, walnut, olive, evening primrose, marrow seed, grapeseed, perilla, sesame, soya bean and sunflower oils. Preferably, the vegetable oil is sunflower oil.

Preferably, the polysaccharide is chosen from gum arabic, guar gum, tara gum, microcrystalline cellulose, and carboxymethylcellulose. More preferably, the polysaccharide is guar gum.

Preferably, the emulsifying agent is chosen from lecithin, sucrose esters, fatty acid mono- and diglycerides, and sorbitan esters. Preferably, the emulsifying agent is chosen from fatty acid monoglycerides.

The invention will be understood more clearly on reading the examples which follow, which are intended to be purely illustrative and do not in any way limit the scope of the protection.

### EXAMPLES

List of the ingredients used:
- NUTRALYS^{®} S85F, Roquette Frères (Lestrem, France)
- NUTRALYS^{®} S85XF, Roquette Frères (Lestrem, France)
- NUTRALYS^{®} W, Roquette Frères (Lestrem, France)
- MPC480, Milk protein concentrate, Protein content 76.7% from Fonterra
- Newfujipro, Soy protein isolate, Protein content about 85%, from Fuji oil
- Sodium metaphosphate (NaₙH₂PₙO₃ₙ₊₁) with n > 14 and Mw > 1,400 available from Taihei Chemical Industrial Co., Ltd.
- Potassium metaphosphate ((KPO₃)ₙ) with Mw = 1,000,000 available from Taihei Chemical Industrial Co., Ltd.

### Example 1a: Prior art - Effect of granulometry on sandy mouthfeel

This example describes the effect of the granulometry of the pea protein isolate on the organoleptic properties of the composition, especially on the sandy mouthfeel.

Two compositions comprising a pea protein isolate, namely NUTRALYS^{®} S85F (Mean diameter 130 microns) or NUTRALYS^{®} S85XF (Mean diameter 40 microns), were prepared. The proportions of the various components (in g) of each composition are presented in the table below.

| | 1 | 2 |
|---|---|---|
| NUTRALYS^{®} S85F | 3 | - |
| NUTRALYS^{®} S85XF | - | 3 |
| Water | 50 | 50 |

The compositions were prepared according to the process below:
- preparing a dry mix of the pea protein isolate,
- mixing said powder with 50 ml of water at room temperature (around 20°C) gently, around 120 rpm for 30 sec, with a spoon in order to dissolve all lumping.

The beverage was rehydrated as described above just before drinking and then given to a panel of experts for sensorial evaluation. Sensory tests were carried out on the compositions in order to evaluate several factors.

For each factor, grades from 1 to 5 were given according to the following scale:
- Sandiness : 1 = Smooth, Less sandy / 5 = Sandy
- Sliminess : 1 = Less slimy, Smooth / 5 = Slimy and sticky
- Saltiness : 1 = Normal clean taste / 5 = Salty taste
- Bitterness : 1 = Normal clean taste / 5 = Bitter taste

The results of the sensory tests are presented in the table below.

| | S85F beverage | S85XF beverage |
|---|---|---|
| Sandiness | 5 | 3 |
| Sliminess | 1 | 1 |
| Saltiness | 1 | 1 |
| Bitterness | 3 | 3 |

The results show that a commercial pea protein isolate with standard granulometry gives a sandy mouthfeel when it is resuspended in tap water. By lowering the granulometry, the sensorial texture is better, but sandiness stays above 3.

### Example 1b: Prior art - Effect of water temperature and dissolution time

This example describes the effect of water temperature and dissolution time on the organoleptic properties of the composition, especially on the sandy mouthfeel.

Five compositions comprising a pea protein isolate, namely NUTRALYS^{®} S85F, were prepared according to the process below:
- preparing a dry mix consisting of 3 g of the pea protein isolate,
- mixing said powder with 50ml of water heated at a temperature of 50°C, 70°C or 90°C, during 30 seconds, 10 minutes or 20 minutes gently, around 120 rpm, with a spoon in order to dissolve all lumping.

The beverage was rehydrated as described above just before drinking and then given to a panel of experts for sensorial evaluation. Sensory tests were carried out on each composition in order to evaluate several factors.

For each factor, grades from 1 to 5 were given according to the following scale:
- Sandiness : 1 = Smooth, Less sandy / 5 = Sandy
- Sliminess : 1 = Less slimy, Smooth / 5 = Slimy and sticky
- Saltiness : 1 = Normal clean taste / 5 = Salty taste
- Bitterness : 1 = Normal clean taste / 5 = Bitter taste

The results of the sensory tests are presented in the table below.

| Water temperature | Dissolution time | Texture & Taste | | | | Total |
|---|---|---|---|---|---|---|
| | | Sandiness | Sliminess | Saltiness | Bitterness | |
| 50°C | 30 s | 5 | 1 | 1 | 3 | 10 |
| 70°C | 30 s | 4 | 1 | 1 | 3 | 9 |
| 90°C | 30 s | 3 | 1 | 1 | 4 | 9 |
| 90°C | 10 min. | 2 | 1 | 1 | 3 | 7 |
| 90°C | 20 min. | 1 | 1 | 1 | 2 | 5 |

The results show that a commercial pea protein isolate may have a good sensorial texture, but use of high temperature water and long dissolution time is mandatory.

### Example 2: Effect of salt addition on sandy mouthfeel - Comparative example

Based on the recipe and process used in Example 1, this example aims to describe the effect of adding a salt on the organoleptic properties of the composition, especially on the sandy mouthfeel.

19 compositions comprising a pea protein isolate, namely NUTRALYS^{®} S85F, and a salt were prepared. The proportions of the various components (in g) of each composition are presented in the tables below.

| | Test No. | | | | | | | | |
|---|---|---|---|---|---|---|---|---|---|
| | 1 | 2 | 3 | 4 | 5 | 6 | 7 | 8 | 9 |
| NUTRALYS S85F | 3 | 3 | 3 | 3 | 3 | 3 | 3 | 3 | 3 |
| Calcium chloride | 0.5 | | | | | | | | |
| Magnesium chloride | | 0.5 | | | | | | | |
| Sodium chloride | | | 0.5 | | | | | | |
| Potassium chloride | | | | 0.5 | | | | | |
| Calcium hydroxide | | | | | 0.5 | | | | |
| Sodium acetate | | | | | | 0.5 | | | |
| Potassium carbonate | | | | | | | 0.5 | | |
| Sodium carbonate | | | | | | | | 0.5 | |
| Citric acid | | | | | | | | | 0.5 |
| Water | 50 | 50 | 50 | 50 | 50 | 50 | 50 | 50 | 50 |

| | Test No. | | | | | | | | |
|---|---|---|---|---|---|---|---|---|---|
| | 10 | 11 | 12 | 13 | 14 | 15 | 16 | 17 | 18 |
| NUTRALYS S85F | 3 | 3 | 3 | 3 | 3 | 3 | 3 | 3 | 3 |
| Sodium citrate | 0.5 | | | | | | | | |
| Potassium citrate | | 0.5 | | | | | | | |
| Dipotassium hydrogenphosphate | | | 0.5 | | | | | | |
| Tri-potassium phosphate | | | | 0.5 | | | | | |
| Tricalcium phosphate | | | | | 0.5 | | | | |
| Sodium metaphosphate | | | | | | 0.5 | | | |
| Potassium gluconate | | | | | | | 0.5 | | |
| Calcium monohydrogenphosphate | | | | | | | | 0.5 | |
| Potassium dihydrogenphosphate | | | | | | | | | 0.5 |
| Water | 50 | 50 | 50 | 50 | 50 | 50 | 50 | 50 | 50 |

The compositions were prepared according to the process below:
- preparing a dry mix of the pea protein isolate,
- mixing said powder with 50 ml of water at room temperature (around 20°C) gently, around 120 rpm for 30 sec, with a spoon in order to dissolve all lumping.

The beverage was rehydrated as described above just before drinking and then given to a panel of experts for sensorial evaluation. Sensory tests were carried out on the compositions in order to evaluate several factors.

For each factor, grades from 1 to 5 were given according to the following scale:
- Sandiness : 1 = Smooth, Less sandy / 5 = Sandy
- Sliminess : 1 = Less slimy, Smooth / 5 = Slimy and sticky
- Saltiness : 1 = Normal clean taste / 5 = Salty taste
- Bitterness : 1 = Normal clean taste / 5 = Bitter taste

The results of the sensory tests are presented in the table below.

| Chemical name of salt (formula) | Taste | | Texture | | Total of texture |
|---|---|---|---|---|---|
| | Saltiness | Bitterness | Sandiness | Sliminess | |
| Calcium chloride (CaCl₂) | 5 | 1 | 5 | 1 | 6 |
| Magnesium chloride (MgCl₂) | 3 | 1 | 5 | 1 | 6 |
| Sodium chloride (NaCl) | 5 | 1 | 5 | 1 | 6 |
| Potassium chloride (KCl) | 3 | 1 | 4 | 1 | 5 |
| Calcium hydroxide (Ca(OH)₂) | 1 | 5 | 5 | 3 | 8 |
| Sodium acetate (C₂H₃NaO₂) | 1 | 1 | 5 | 1 | 6 |
| Potassium carbonate (K₂CO₃) | 3 | 2 | 5 | 1 | 6 |
| Sodium carbonate (Na₂CO₃) | 3 | 2 | 5 | 1 | 6 |
| Citric acid (C₆H₈O₇) | 1 | 1 | 5 | 1 | 6 |
| Sodium citrate (Na₃C₆H₅O₇) | 1 | 1 | 4 | 1 | 5 |
| Potassium citrate (C₆H₅K₃O₇) | 1 | 1 | 4 | 1 | 5 |
| Dipotassium hydrogenphosphate (K₂HPO₄) | 1 | 1 | 4 | 1 | 5 |
| Tri-potassium phosphate (K₃PO₄) | 1 | 3 | 2 | 3 | 5 |
| Tricalcium phosphate (Ca₃(PO₄)₂) | 1 | 1 | 5 | 1 | 6 |
| Sodium metaphosphate (NaₙH₂PₙO₃ₙ₊₁) | 1 | 1 | 3 | 1 | 4 |
| Potassium gluconate (HOCH₂(CHOH)₄COOK) | 2 | 1 | 5 | 1 | 6 |
| Calcium monohydrogenphosphate (CaHPO₄ • 2 H₂O) | 1 | 1 | 5 | 1 | 6 |
| Potassium dihydrogenphosphate (KH₂PO₄) | 1 | 1 | 5 | 1 | 6 |

The results show that addition of sodium metaphosphate in a commercial pea protein isolate of standard granulometry lowers the sandy mouthfeel when it is resuspended in tap water. In fact, the sandiness mouthfeel is lower than that obtained with a pea protein isolate of low granulometry (NUTRALYS^{®} S85XF) according to example 1a.

### Example 3: Effect of type of phosphate salt - Comparison of potassium metaphosphate with other salts studied as comparative examples

Based on the recipe and process used in Examples above, this example aims to describe the effect of adding a salt on the organoleptic properties of the composition, especially on the sandy mouthfeel.

11 compositions comprising a pea protein isolate, namely NUTRALYS^{®} S85F, and a salt were prepared. The proportions of the various components (in g) of each composition are presented in the table below.

| | Test No. | | | | | | | | | | |
|---|---|---|---|---|---|---|---|---|---|---|---|
| | 1 | 2 | 3 | 4 | 5 | 6 | 7 | 8 | 9 | 10 | 11 |
| NUTRALYS S85F | 3 | 3 | 3 | 3 | 3 | 3 | 3 | 3 | 3 | 3 | 3 |
| Potassium metaphosphate | 0.5 | | | | | | | | | | |
| Potassium tripolyphosphate | | 0.5 | | | | | | | | | |
| Potassium pyrophosphate | | | 0.5 | | | | | | | | |
| Sodium tripolyphosphate | | | | 0.5 | | | | | | | |
| Trisodium pyrophosphate | | | | | 0.5 | | | | | | |
| Disodium dihydrogen pyrophosphate | | | | | | 0.5 | | | | | |
| Trisodium phosphate | | | | | | | 0.5 | | | | |
| Disodium hydrogen phosphate | | | | | | | | 0.5 | | | |
| Sodium dihydrogenphosphate | | | | | | | | | 0.5 | | |
| Tri-potassium phosphate | | | | | | | | | | 0.5 | |
| Sodium metaphosphate | | | | | | | | | | | 0.5 |
| Water | 50 | 50 | 50 | 50 | 50 | 50 | 50 | 50 | 50 | 50 | 50 |

The compositions were prepared according to the process below:
- preparing a dry mix of the pea protein isolate,
- mixing said powder with 50 ml of water at room temperature (around 20°C) gently, around 120 rpm for 30 sec, with a spoon in order to dissolve all lumping.

The beverage was rehydrated as described above just before drinking and then given to a panel of experts for sensorial evaluation. Sensory tests were carried out on the compositions in order to evaluate several factors.

For each factor, grades from 1 to 5 were given according to the following scale:
- Sandiness : 1 = Smooth, Less sandy / 5 = Sandy
- Bitterness : 1 = Normal clean taste / 5 = Bitter taste
- Solubility : 1 = Soluble. Dissolves easily / 5 = Less soluble.
- Acidity : 1 = Normal clean taste / 5 = Acid taste

The results of the sensory tests are presented in the table below.

| Name of phosphate | Taste | | Texture | | Total |
|---|---|---|---|---|---|
| | Bitternes s | Acidity | Sandiness | Solubility | |
| Potassium metaphosphate | 1 | 1 | 1 | 2 | 5 |
| Potassium tripolyphosphate | 1 | 1 | 3 | 1 | 6 |
| Potassium pyrophasphate | 1 | 1 | 4 | 1 | 7 |
| Sodium tripolyphosphate | 1 | 2 | 4 | 1 | 8 |
| Trisodium pyrophosphate | 1 | 1 | 5 | 3 | 10 |
| Disodium dihydrogen pyrophosphate | 1 | 2 | 5 | 1 | 9 |
| Trisodium phosphate | 2 | 1 | 2 | 2 | 7 |
| Disodium hydrogen phosphate | 1 | 1 | 5 | 3 | 10 |
| Sodium dihydrogenphosphate | 1 | 2 | 5 | 1 | 9 |
| Tri-potassium phosphate | 3 | 1 | 2 | 1 | 7 |
| Sodium metaphosphate | 1 | 1 | 3 | 1 | 6 |

The results clearly show that addition of potassium metaphosphate gives the best results when used with a commercial pea protein isolate of standard granulometry, as it gives the lowest sandy mouthfeel when it is resuspended in tap water. In fact, the sandiness mouthfeel is lower than that obtained with a pea protein isolate of low granulometry (NUTRALYS^{®} S85XF) according to example 1a.

### Example 4: Effect of amount of potassium metaphosphate

Based on the recipe and process used in Examples above, this example aims to describe the effect of the amount of potassium metaphosphate on the organoleptic properties of the composition, especially on the sandy mouthfeel.

11 compositions comprising a pea protein isolate, namely NUTRALYS^{®} S85F, and potassium metaphosphate were prepared. The proportions of the various components (in g) of each composition are presented in the tables below.

| | Amount of potassium metaphosphate [% by weight based on the dry weight of the composition] | | | | | | |
|---|---|---|---|---|---|---|---|
| | 45% | 33% | 8% | 2% | 0.4% | 0.2% | 0.08% |
| NUTRALYS S85F (g) | 3 | 3 | 3 | 3 | 3 | 3 | 3 |
| Potassium metaphosphate (g) | 2.5 | 1.5 | 0.25 | 0.0625 | 0.0125 | 0.0075 | 0.0025 |
| Water (g) | 50 | 50 | 50 | 50 | 50 | 50 | 50 |

| 0.04% | | 0.025% | | 0.008% | | 0.004% | |
|---|---|---|---|---|---|---|---|
| NUTRALYS S85F (g) | 3 | | 3 | | 3 | | 3 |
| Potassium metaphosphate (g) | 0.00125 | | 0.00075 | | 0.00025 | | 0.000125 |
| Water (g) | 50 | | 50 | | 50 | | 50 |

The compositions were prepared according to the process below:
- preparing a dry mix of the pea protein isolate,
- mixing said powder with 50 ml of water at room temperature (around 20°C) gently, around 120 rpm for 30 sec, with a spoon in order to dissolve all lumping.

The beverage was rehydrated as described above just before drinking and then given to a panel of experts for sensorial evaluation. Sensory tests were carried out on the compositions in order to evaluate several factors.

For each factor, grades from 1 to 5 were given according to the following scale:
- Sandiness : 1 = Smooth, Less sandy / 5 = Sandy
- Bitterness : 1 = Normal clean taste / 5 = Bitter taste
- Solubility : 1 = Soluble. Dissolves easily / 5 = Less soluble

The results of the sensory tests are presented in the table below.

| | | Taste | Texture | | Total |
|---|---|---|---|---|---|
| | | Bitterness | Sandiness | Solubility | |
| | 45% | 2 | 1 | 3 | 5 |
| | 33% | 1 | 1 | 2 | 4 |
| | 8% | 1 | 1 | 1 | 3 |
| | 2% | 1 | 1 | 1 | 3 |
| Amount of potassium metaphosphate [% by weight based on the dry weight of the composition] | 0.4% | 1 | 1 | 1 | 3 |
| | 0.2% | 1 | 1 | 1 | 3 |
| | 0.08% | 1 | 1 | 1 | 3 |
| | 0.04% | 1 | 1 | 1 | 3 |
| | 0.025% | 1 | 1 | 1 | 3 |
| | 0.008% | 1 | 2 | 1 | 4 |
| | 0.004% | 1 | 3 | 1 | 5 |

### Example 5: Synergy of phosphate salt dosage

Based on the recipe and process used in Examples above, this example aims to describe the effect of the combination of potassium metaphosphate and another phosphate salt on the organoleptic properties of the composition, especially on the sandy mouthfeel. 11 compositions comprising a pea protein isolate, namely NUTRALYS^{®} S85F, potassium metaphosphate and another phosphate salt were prepared. The proportions of the various components (in g) of each composition are presented in the table below.

| | Test No. | | | | | | |
|---|---|---|---|---|---|---|---|
| | 1 | 2 | 3 | 4 | 5 | 6 | 7 |
| NUTRALYS S85F | 3 | 3 | 3 | 3 | 3 | 3 | 3 |
| Sodium metaphosphate | 0.2 | | | | | | |
| Tri-potassium phosphate | | 0.2 | | | | | |
| Trisodium phosphate | | | 0.2 | | | | |
| Sodium tripolyphosphate | | | | 0.2 | | | |
| Potassium pyrophosphate | | | | | 0.2 | | |
| Potassium tripolyphosphate | | | | | | 0.2 | |
| Potassium metaphosphate | 0.2 | 0.2 | 0.2 | 0.2 | 0.2 | 0.2 | 0.4 |
| Water | 50 | 50 | 50 | 50 | 50 | 50 | 50 |

The compositions were prepared according to the process below:
- preparing a dry mix of the pea protein isolate,
- mixing said powder with 50 ml of water at room temperature (around 20°C) gently, around 120 rpm for 30 sec, with a spoon in order to dissolve all lumping.

The beverage was rehydrated as described above just before drinking and then given to a panel of experts for sensorial evaluation. Sensory tests were carried out on the compositions in order to evaluate several factors.

For each factor, grades from 1 to 5 were given according to the following scale:
- Sandiness : 1 = Smooth, Less sandy / 5 = Sandy
- Bitterness : 1 = Normal clean taste / 5 = Bitter taste
- Solubility : 1 = Soluble. Dissolves easily / 5 = Less soluble.
- Acidity : 1 = Normal clean taste / 5 = Acid taste

The results of the sensory tests are presented in the table below.

| Phosphates [Combination use] | | Taste | | Texture | | Total |
|---|---|---|---|---|---|---|
| | | Bitterness | Acidity | Sandiness | Solubility | |
| Potassium metaphosphate + | Sodium metaphosphate | 1 | 1 | 1 | 1 | 4 |
| | Tri-potassium phosphate | 2 | 1 | 4 | 1 | 8 |
| | Trisodium phosphate | 3 | 1 | 2 | 3 | 9 |
| | Sodium tripolyphosphate | 1 | 1 | 3 | 1 | 6 |
| | Potassium pyrophosphate | 1 | 1 | 4 | 1 | 7 |
| | Potassium tripolyphosphate | 1 | 1 | 5 | 1 | 8 |
| Potassium metaphosphate only | | 1 | 1 | 3 | 2 | 7 |

Results clearly show a synergy for the combination of potassium metaphosphate and sodium metaphosphate.

### Example 6: Effect of the amount of potassium metaphosphate based on the total amount of sodium metaphosphate and potassium metaphosphate

Based on the recipe and process used in Examples above, this example aims to describe the effect of the weight percentage of potassium metaphosphate based on the total weight of sodium metaphosphate and potassium metaphosphate on the organoleptic properties of the composition, especially on the sandy mouthfeel.

Compositions comprising a pea protein isolate, namely NUTRALYS^{®} S85F, potassium metaphosphate and sodium metaphosphate were prepared. The proportions of the various components (in g) of each composition are presented in the table below.

| | Weight percentage of potassium metaphosphate based on the total weight of sodium metaphosphate and potassium metaphosphate | | | | | | | |
|---|---|---|---|---|---|---|---|---|
| | 100% | 80% | 50% | 35% | 20% | 10% | 2% | 1% |
| NUTRALYS S85F | 3 | 3 | 3 | 3 | 3 | 3 | 3 | 3 |
| Vegetable powder | 0.5 | 0.5 | 0.5 | 0.5 | 0.5 | 0.5 | 0.5 | 0.5 |
| Fruit powder | 0.5 | 0.5 | 0.5 | 0.5 | 0.5 | 0.5 | 0.5 | 0.5 |
| Sodium metaphosphate | 0.000 | 0.075 | 0.188 | 0.244 | 0.300 | 0.338 | 0.368 | 0.371 |
| Potassium metaphosphate | 0.375 | 0.300 | 0.188 | 0.131 | 0.075 | 0.038 | 0.008 | 0.004 |
| Water | 50 | 50 | 50 | 50 | 50 | 50 | 50 | 50 |

The compositions were prepared according to the process below:
- preparing a dry mix of the pea protein isolate,
- mixing said powder with 50 ml of water at room temperature (around 20°C) gently, around 120 rpm for 30 sec, with a spoon in order to dissolve all lumping.

The beverage was rehydrated as described above just before drinking and then given to a panel of experts for sensorial evaluation. Sensory tests were carried out on the compositions in order to evaluate several factors.

For each factor, grades from 1 to 5 were given according to the following scale:
- Sandiness : 1 = Smooth, Less sandy / 5 = Sandy
- Body : 1 = Fluid texture. No body texture / 5= Body, heavy texture

The results of the sensory tests are presented in the table below.

| Proportion of potassium metaphosphate | Texture | | Total |
|---|---|---|---|
| | Sandiness | Body | |
| 100% | 3 | 1 | 4 |
| 80% | 2 | 1 | 3 |
| 50% | 1 | 1 | 2 |
| 35% | 1 | 1 | 2 |
| 20% | 1 | 1 | 2 |
| 10% | 1 | 1 | 2 |
| 2% | 1 | 2 | 3 |
| 1% | 2 | 3 | 5 |

Results clearly show that the texture is best when potassium metaphosphate represents from 2 to 80% by weight of the total weight of potassium metaphosphate and sodium metaphosphate in the composition.

### Example 7: Effect of amount of potassium metaphosphate and sodium metaphosphate (weight ratio 50/50)

Based on the recipe and process used in Examples above, this example aims to describe the effect of the weight percentage of potassium metaphosphate and sodium metaphosphate based on the dry weight of the composition (with a 50/50 weight ratio of potassium metaphosphate to sodium metaphosphate) on the organoleptic qualities of the composition, especially on the sandy mouthfeel.

Compositions comprising a pea protein isolate, namely NUTRALYS^{®} S85F, sodium metaphosphate and potassium metaphosphate were prepared. The proportions of the various components (in g) of each composition are presented in the tables below.

| | % by weight of sodium metaphosphate and potassium metaphosphate based on the dry weight of the composition | | | | | | |
|---|---|---|---|---|---|---|---|
| | 10% | 5% | 2% | 1% | 0.50% | 0.30% | 0.20% |
| NUTRALYS S85F | 3 | 3 | 3 | 3 | 3 | 3 | 3 |
| Vegetable powder | 0.5 | 0.5 | 0.5 | 0.5 | 0.5 | 0.5 | 0.5 |
| Fruit powder | 0.5 | 0.5 | 0.5 | 0.5 | 0.5 | 0.5 | 0.5 |
| Sodium metaphosphate | 2.5 | 1.25 | 0.5 | 0.25 | 0.125 | 0.075 | 0.05 |
| Potassium metaphosphate | 2.5 | 1.25 | 0.5 | 0.25 | 0.125 | 0.075 | 0.05 |
| Water | 50 | 50 | 50 | 50 | 50 | 50 | 50 |

| | % by weight of sodium metaphosphate and potassium metaphosphate based on the dry weight of the composition | | | |
|---|---|---|---|---|
| | 0.10% | 0.05% | 0.025% | 0.0125% |
| NUTRALYS S85F | 3 | 3 | 3 | 3 |
| Vegetable powder | 0.5 | 0.5 | 0.5 | 0.5 |
| Fruit powder | 0.5 | 0.5 | 0.5 | 0.5 |
| Sodium metaphosphate | 0.025 | 0.0125 | 0.00625 | 0.003125 |
| Potassium metaphosphate | 0.025 | 0.0125 | 0.00625 | 0.003125 |
| Water | 50 | 50 | 50 | 50 |

The compositions were prepared according to the process below:
- preparing a dry mix of the pea protein isolate,
- mixing said powder with 50 ml of water at room temperature (around 20°C) gently, around 120 rpm for 30 sec, with a spoon in order to dissolve all lumping.

The beverage was rehydrated as described above just before drinking and then given to a panel of experts for sensorial evaluation. Sensory tests were carried out on the compositions in order to evaluate several factors.

For each factor, grades from 1 to 5 were given according to the following scale:
- Sandiness : 1 = Smooth, Less sandy / 5 = Sandy
   Sliminess : 1 = Less slimy, Smooth / 5 = Slimy and sticky

The results of the sensory tests are presented in the table below.

| % by weight of sodium metaphosphate and potassium metaphosphate | Texture | | Taste | Total |
|---|---|---|---|---|
| | Sandiness | Sliminess | | |
| 10% | 1 | 3 | Mineral taste | 4 |
| 5% | 1 | 1 | OK | 2 |
| 2% | 1 | 1 | OK | 2 |
| 1% | 1 | 1 | OK | 2 |
| 0.5% | 1 | 1 | OK | 2 |
| 0.3% | 1 | 1 | OK | 2 |
| 0.2% | 1 | 1 | OK | 2 |
| 0.1% | 1 | 1 | OK | 2 |
| 0.05% | 1 | 1 | OK | 2 |
| 0.025% | 1 | 1 | OK | 2 |
| 0.0125% | 3 | 1 | OK | 4 |

Results clearly show that the amount of such mixture can be comprised between 0.025% and 5% by weight based on the dry weight of the composition.

### Example 8: Effect of the combination of sodium metaphosphate and potassium metaphosphate on other plant protein isolates - Not according to the invention

Based on the recipe and process used in Examples above, this example aims to describe that the combination of sodium metaphosphate and potassium metaphosphate can also be used to improve the organoleptic qualities, especially the sandy mouthfeel, of compositions comprising other protein isolates.

Compositions comprising protein isolates, potassium metaphosphate and sodium metaphosphate were prepared. The proportions of the various components (in g) of each composition are presented in the table below.

| | Soy-1 | Soy-2 | Wheat-1 | Wheat-2 | Milk-1 | Milk-2 |
|---|---|---|---|---|---|---|
| Soy protein | 3 | 3 | | | | |
| NUTRALYS W | | | 3 | 3 | | |
| Milk protein | | | | | 3 | 3 |
| Sodium metaphosphate | | 0.07 | | 0.07 | | 0.07 |
| Potassium metaphosphate | | 0.07 | | 0.07 | | 0.07 |
| Vegetable powder | 0.5 | 0.5 | 0.5 | 0.5 | 0.5 | 0.5 |
| Fruit powder | 0.5 | 0.5 | 0.5 | 0.5 | 0.5 | 0.5 |
| Water | 50 | 50 | 50 | 50 | 50 | 50 |

The compositions were prepared according to the process below:
- preparing a dry mix of the pea protein isolate,
- mixing said powder with 50 ml of water at room temperature (around 20°C) gently, around 120 rpm for 30 sec, with a spoon in order to dissolve all lumping.

The beverage was rehydrated as described above just before drinking and then given to a panel of experts for sensorial evaluation. Sensory tests were carried out on the compositions in order to evaluate several factors.

For each factor, grades from 1 to 5 were given according to the following scale:
- Sandiness : 1 = Smooth, Less sandy / 5 = Sandy
- Sliminess : 1 = Less slimy, Smooth / 5 = Slimy and sticky
- Solubility : 1= Soluble. Dissolve easily / 5 = Less soluble.

The results of the sensory tests are presented in the table below.

| Test name | Texture | | | Total |
|---|---|---|---|---|
| | Sandiness | Solubility | Sliminess | |
| Soy-1 | 3 | 2 | 1 | 6 |
| Soy-2 | 1 | 2 | 1 | 4 |
| Wheat-1 | 2 | 5 | 2 | 9 |
| Wheat-2 | 1 | 4 | 2 | 7 |
| Milk-1 | 2 | 2 | 1 | 5 |
| Milk-2 | 1 | 1 | 1 | 3 |

Results clearly show that a mixture of sodium metaphosphate and potassium metaphosphate can be used to improve the organoleptic properties of compositions comprising soy, wheat or milk proteins.

## Claims

1. A rehydratable food composition **characterized in that** it comprises pea proteins, and a potassium metaphosphate.

2. The composition of Claim 1 **characterized in that** the amount of potassium metaphosphate in the composition is from 0.008% to 33%, preferably from 0.01% to 20%, more preferably from 0.025 to 8%, by weight based on the dry weight of the composition.

3. The composition of Claim 1 or 2 **characterized in that** the composition further comprises a sodium metaphosphate.

4. The composition of Claim 3 **characterized in that** the total amount of sodium metaphosphate and potassium metaphosphate in the composition is from 0.01% to 8%, preferably from 0.02% to 6%, more preferably from 0.025% to 5%, by weight based on the dry weight of the composition.

5. The composition of Claim 3 or 4, wherein the weight percentage of potassium metaphosphate based on the total weight of potassium metaphosphate and sodium metaphosphate in the composition is from 2% to 80%, preferably from 5% to 60%, even more preferably from 10% to 50%.

6. A process for preparing a rehydratable food composition comprising mixing pea proteins, and a potassium metaphosphate.

7. The process of Claim 6, **characterized in that** the process comprises mixing pea proteins, and a combination of a sodium metaphosphate and a potassium metaphosphate.

8. The process of Claim 6 or 7 wherein the composition obtained by mixing the pea proteins and the potassium metaphosphate or the combination of sodium metaphosphate and potassium metaphosphate is a solid composition, in particular a dry powder mix.

9. The process of Claim 6 or 7 wherein the composition obtained by mixing the pea proteins and the potassium metaphosphate or the combination of sodium metaphosphate and potassium metaphosphate is an aqueous composition, in particular an aqueous solution or suspension.

10. The process of Claim 9, wherein the aqueous composition is further heat-sterilized and dried.

11. Use of the composition of any one of Claims 1 to 5 or obtainable by the process of any one of Claims 6 to 10 in the preparation of a food composition, feed composition or pharmaceutical composition.

## Patentansprüche

1. Rehydrierbare Lebensmittelzusammensetzung, **dadurch gekennzeichnet, dass** sie Erbsenproteine und ein Kaliummetaphosphat umfasst.

2. Zusammensetzung nach Anspruch 1, **dadurch gekennzeichnet, dass** die Menge an Kaliummetaphosphat in der Zusammensetzung von 0,008 Gew.-% bis 33 Gew.-%, bevorzugt von 0,01 Gew.-% bis 20 Gew.-%, stärker bevorzugt von 0,025 bis 8 Gew.-%, bezogen auf das Trockengewicht der Zusammensetzung, beträgt.

3. Zusammensetzung nach Anspruch 1 oder 2, **dadurch gekennzeichnet, dass** die Zusammensetzung ferner ein Natriummetaphosphat umfasst.

4. Zusammensetzung nach Anspruch 3, **dadurch gekennzeichnet, dass** die Gesamtmenge an Natriummetaphosphat und Kaliummetaphosphat in der Zusammensetzung von 0,01 Gew.-% bis 8 Gew.-%, bevorzugt von 0,02 Gew.-% bis 6 Gew.- %, stärker bevorzugt von 0,025 Gew.-% bis 5 Gew.-%, bezogen auf das Trockengewicht der Zusammensetzung, beträgt.

5. Zusammensetzung nach Anspruch 3 oder 4, wobei der Gewichtsanteil an Kaliummetaphosphat, bezogen auf das Gesamtgewicht an Kaliummetaphosphat und Natriummetaphosphat in der Zusammensetzung, von 2 Gew.-% bis 80 Gew.-%, bevorzugt von 5 Gew.-% bis 60 Gew.-%, noch stärker bevorzugt von 10 Gew.-% bis 50 Gew.-% beträgt.

6. Verfahren zur Herstellung einer rehydrierbaren Lebensmittelzusammensetzung, umfassend Mischen von Erbsenproteinen und einem Kaliummetaphosphat.

7. Verfahren nach Anspruch 6, **dadurch gekennzeichnet, dass** das Verfahren Mischen von Erbsenproteinen und einer Kombination aus einem Natriummetaphosphat und einem Kaliummetaphosphat umfasst.

8. Verfahren nach Anspruch 6 oder 7, wobei die Zusammensetzung, welche durch Mischen der Erbsenproteine und des Kaliummetaphosphats oder der Kombination von Natriummetaphosphat und Kaliummetaphosphat erhalten wird, eine feste Zusammensetzung ist, insbesondere eine trockene Pulvermischung.

9. Verfahren nach Anspruch 6 oder 7, wobei die Zusammensetzung, welche durch Mischen der Erbsenproteine und des Kaliummetaphosphats oder der Kombination von Natriummetaphosphat und Kaliummetaphosphat erhalten wird, eine wässrige Zusammensetzung ist, insbesondere eine wässrige Lösung oder Suspension.

10. Verfahren nach Anspruch 9, wobei die wässrige Zusammensetzung ferner hitzesterilisiert und getrocknet wird.

11. Verwendung der Zusammensetzung nach einem der Ansprüche 1 bis 5 oder erhältlich durch das Verfahren nach einem der Ansprüche 6 bis 10 in der Herstellung einer Lebensmittelzusammensetzung, Futterzusammensetzung oder pharmazeutischen Zusammensetzung.

## Revendications

1. Composition alimentaire réhydratable **caractérisée en ce qu'**elle comprend des protéines de pois, et un métaphosphate de potassium.

2. Composition selon la revendication 1, **caractérisée en ce que** la quantité de métaphosphate de potassium dans la composition est de 0,008 % à 33 %, de préférence de 0,01 % à 20 %, plus préférentiellement de 0,025 à 8 % en poids par rapport au poids sec de la composition.

3. Composition selon la revendication 1 ou 2 **caractérisée en ce que** la composition comprend en outre un métaphosphate de sodium.

4. Composition selon la revendication 3 **caractérisée en ce que** la quantité totale de métaphosphate de sodium et de métaphosphate de potassium dans la composition est de 0,01 % à 8 %, de préférence de 0,02 % à 6 %, plus préférentiellement de 0,025 % à 5 % en poids par rapport au poids sec de la composition.

5. Composition selon la revendication 3 ou 4, dans laquelle le pourcentage en poids de métaphosphate de potassium sur la base du poids total de métaphosphate de potassium et de métaphosphate de sodium dans la composition est de 2 % à 80 %, de préférence de 5 % à 60 %, encore plus préférentiellement de 10 % à 50 %.

6. Procédé de préparation d'une composition alimentaire réhydratable comprenant le mélange de protéines de pois et d'un métaphosphate de potassium.

7. Procédé selon la revendication 6, **caractérisé en ce que** le procédé comprend le mélange de protéines de pois, et d'une combinaison d'un métaphosphate de sodium et d'un métaphosphate de potassium.

8. Procédé selon la revendication 6 ou 7 dans lequel la composition obtenue par le mélange des protéines de pois et du métaphosphate de potassium ou de la combinaison de métaphosphate de sodium et de métaphosphate de potassium est une composition solide, en particulier un mélange de poudre sèche.

9. Procédé selon la revendication 6 ou 7 dans lequel la composition obtenue par le mélange des protéines de pois et du métaphosphate de potassium ou de la combinaison de métaphosphate de sodium et de métaphosphate de potassium est une composition aqueuse, en particulier une solution ou suspension aqueuse.

10. Procédé selon la revendication 9, dans lequel la composition aqueuse est en outre stérilisée à la chaleur et séchée.

11. Utilisation de la composition selon l'une quelconque des revendications 1 à 5 ou pouvant être obtenue par le procédé selon l'une quelconque des revendications 6 à 10 dans la préparation d'une composition alimentaire, d'une composition d'aliments pour animaux ou d'une composition pharmaceutique.
